# EUROPEAN PATENT SPECIFICATION

(11) **EP 3 716 854 B1**
(45) Date of publication and mention of the grant of the patent: **28.05.2025**
(21) Application number: 18821828.3
(22) Date of filing: 29.11.2018
(51) Int. Cl.: A61B 8/00, A61B 8/08

(54) **SYSTEM AND METHOD FOR MOUNTING AN ULTRASOUND TRANSDUCER ON A NEEDLE**
SYSTEM UND VERFAHREN ZUR MONTAGE EINES ULTRASCHALLWANDLERS AUF EINER NADEL
SYSTÈME ET PROCÉDÉ DE MONTAGE D'UN TRANSDUCTEUR ULTRASONORE SUR UNE AIGUILLE

(30) Priority: 29.11.2017 US 201762591995 P
(43) Date of publication of application: 07.10.2020
(73) Proprietor: Avent, Inc., Alpharetta, GA 30004 (US)
(72) Inventor: KHALAJ, Steve S., Alpharetta Georgia 30004 (US); SHAHRIARI, Shirzad, Alpharetta Georgia 30004 (US)
(74) Representative: Dehns
(86) International application number: PCT/US2018/062948
(87) International publication number: WO 2019/108722

(56) References cited:
- WO-A1-97/29682
- DE-A1- 19 750 335
- US-A- 5 779 643
- US-A1- 2004 186 368
- US-A1- 2007 129 628
- US-A1- 2011 184 313
- US-A1- 2013 041 259
- US-A1- 2013 109 974
- US-A1- 2014 024 945
- US-A1- 2015 374 348
- US-A1- 2016 074 021
- US-A1- 2016 287 223

## Description

### RELATED APPLICATIONS

The present invention claims priority to U.S. Provisional Application No.: 62/591,995 filed on November 29, 2017.

### FIELD OF THE INVENTION

The present invention relates to generally to medical imaging, and more particularly, to systems and methods for mounting an ultrasound transducer of an ultrasound imaging system on a needle to be inserted into a patient.

### BACKGROUND

Detection of anatomical objects using medical imaging is an essential step for many medical procedures, such as regional anesthesia nerve blocks, and is becoming the standard in clinical practice to support diagnosis, patient stratification, therapy planning, intervention, and/or follow-up. Various systems based on traditional approaches exist for anatomical detection and tracking in medical images, such as computed tomography (CT), magnetic resonance (MR), ultrasound, and fluoroscopic images.

For example, ultrasound imaging systems utilize sound waves with frequencies higher than the upper audible limit of human hearing. Further, ultrasound imaging systems are widely used in medicine to perform both diagnosis and therapeutic procedures. In such procedures, sonographers perform scans of a patient using a hand-held probe or transducer that is placed directly on and moved over the patient.

Ultrasonic transducers come in a variety of different shapes and sizes for use in making cross-sectional images of various parts of the body. The transducer may be passed over the surface and in contact with the body or may be inserted into a patient. Oftentimes, however, it can be difficult to locate the transducer within a patient at a desired target site. In addition, it can be challenging to maintain the transducer at a certain angle that allows for optimal signal processing of the sound waves.

Accordingly, the present disclosure is directed to a system and method for mounting an ultrasound transducer on a needle to be inserted into a patient that addresses the aforementioned issues.

A prior art needle assembly, having the features of the preamble of claim 1, is disclosed in US 2013/041259 A1. Further prior art needle assemblies are disclosed in WO 97/29682 A1, DE 197 50 335 A1, US 2016/287223 A1, US 2014/024945 A1, US 2013/109974 A1, US 2007/129628 A1 and US 5 779 643 A.

### SUMMARY OF THE INVENTION

Objects and advantages of the invention will be set forth in part in the following description, or may be obvious from the description, or may be learned through practice of the invention.

In one aspect, the present invention is directed to a needle assembly for an ultrasound imaging system, in accordance with claim 1. In addition, in certain embodiments, the predetermined angle may be adjustable.

In additional embodiments, the ultrasound transducer may be a flat unidirectional transducer. Alternatively, the ultrasound transducer may include a cylindrical transducer. In such embodiments, the distal end of the needle may include a small outer diameter than the proximal end. Thus, the cylindrical transducer fits around the small outer diameter of the distal end of the needle.

In further embodiments, which are not claimed, a portion of the distal end of the needle may extend beyond the cylindrical transducer. In such embodiments, the needle assembly may include a needle tip secured to the portion of the distal end of the needle that extends beyond the cylindrical transducer.

In yet another embodiment, the outer wall may include one or more grooves for receiving one or more wires associated with the ultrasound transducer. In such embodiments, the groove(s) may be embedded within the outer wall of the needle.

In another aspect, the present invention is directed to a method for mounting an ultrasound transducer of an ultrasound imaging system on a needle to be inserted into a patient, in accordance with claim 8. In some embodiments, the method may also include adjusting an angle of the angled bottom surface.

In still another embodiment, not falling within the scope of the invention, the step of machining the outer wall of the needle to accommodate the ultrasound transducer may include removing a cylindrical portion of the outer wall of the needle to decrease the outer diameter of the needle and placing the ultrasound transducer around the reduced outer diameter.

In additional embodiments, the method may include forming one or more grooves in the outer wall of the needle and routing one or more wires associated with the ultrasound transducer through the one or more grooves. It should also be understood that the method may further include any of the additional steps and/or features as described herein.

These and other features, aspects and advantages of the present invention will become better understood with reference to the following description and appended claims. The accompanying drawings, which are incorporated in and constitute a part of this specification, illustrate embodiments of the invention and, together with the description, serve to explain the principles of the invention.

### BRIEF DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1 illustrates a perspective view of one embodiment of an imaging system according to the present disclosure;
FIG. 2 illustrates a block diagram one of embodiment of a controller of an imaging system according to the present disclosure;
FIG. 3 illustrates a cross-sectional view of one example of a needle assembly, outside the wording of the claims, particularly illustrating an ultrasound transducer mounted to an outer wall of a needle;
FIG. 4 illustrates a detailed, side view of one embodiment of a needle assembly according to the present invention, particularly illustrating an ultrasound transducer mounted to an angled bottom surface of a cavity within an outer wall of a needle;
FIG. 5 illustrates a cross-sectional view of an example of a needle assembly outside the wording of the claims, particularly illustrating an ultrasound transducer mounted to a flap formed of an outer wall of a needle;
FIG. 6 illustrates a top view of the needle assembly of FIG. 5;
FIG. 7 illustrates a perspective view of yet another example of a needle assembly, outside the wording of the claims, particularly illustrating an ultrasound transducer mounted around an outer wall of a needle;
FIG. 8 illustrates a cross-sectional view of still another example of a needle assembly, outside the wording of the claims, particularly illustrating an ultrasound transducer mounted around an outer wall of a needle with a needle tip mounted adjacent to the transducer; and
FIG. 9 illustrates a flow diagram of one embodiment of a method for mounting an ultrasound transducer of an ultrasound imaging system on a needle to be inserted into a patient according to the present disclosure.

### DETAILED DESCRIPTION OF THE INVENTION

Reference will now be made in detail to one or more embodiments of the invention, examples of the invention, examples of which are illustrated in the drawings. Each example and embodiment is provided by way of explanation of the invention, and is not meant as a limitation of the invention. For example, features illustrated or described as part of one embodiment may be used with another embodiment to yield still a further embodiment, as far as this embodiment falls within the scope of the appended claims.

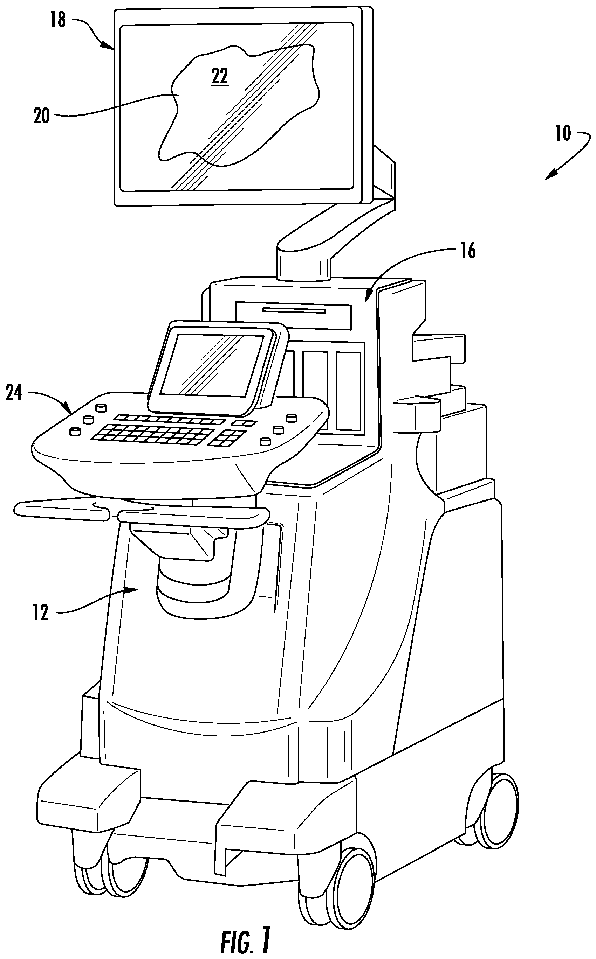

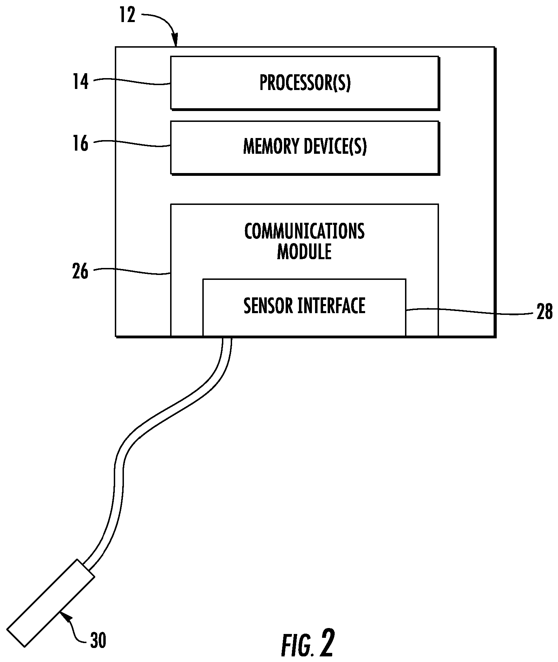

Referring now to the drawings, FIGS. 1 and 2 illustrate a medical imaging system 10 for scanning, identifying, and navigating anatomical objects of a patient according to the present disclosure. As used herein, the anatomical object(s) 22 and surrounding tissue described herein may include any anatomical structure and/or surrounding tissue of a patient. For example, in one embodiment, the anatomical object(s) 22 may include an interscalene brachial plexus of the patient, which generally corresponds to the network of nerves running from the spine, formed by the anterior rami of the lower four cervical nerves and first thoracic nerve. As such, the surrounding tissue of the brachial plexus generally corresponds to the sternocleidomastoid muscle, the middle scalene muscle, the anterior scalene muscle, and/or similar. **It** should be understood, however, that the system and method of the present disclosure may be further used for any variety of medical procedures involving any anatomical structure in addition to those relating to the brachial plexus. For example, the anatomical object(s) 22 may include upper and lower extremities, as well as compartment blocks. More specifically, in such embodiments, the anatomical object(s) 22 of the upper extremities may include interscalene muscle, supraclavicular muscle, infraclavicular muscle, and/or axillary muscle nerve blocks, which all block the brachial plexus (a bundle of nerves to the upper extremity), but at different locations. Further, the anatomical object(s) 22 of the lower extremities may include the lumbar plexus, the fascia Iliac, the femoral nerve, the sciatic nerve, the abductor canal, the popliteal, the saphenous (ankle), and/or similar. **In** addition, the anatomical object(s) 22 of the compartment blocks may include the intercostal space, transversus abdominus plane, and thoracic paravertebral space, and/or similar.

More specifically, as shown, the imaging system 10 may correspond to an ultrasound imaging system or any other suitable imaging system that can benefit from the present technology. Thus, as shown, the imaging system 10 may generally include a controller 12 having one or more processor(s) 14 and associated memory device(s) 16 configured to perform a variety of computer-implemented functions (e.g., performing the methods and the like and storing relevant data as disclosed herein), as well as a user display 18 configured to display an image 20 of an anatomical object 22 to an operator. In addition, the imaging system 10 may include a user interface 24, such as a computer and/or keyboard, configured to assist a user in generating and/or manipulating the user display 18.

Additionally, as shown in FIG. 2, the processor(s) 14 may also include a communications module 26 to facilitate communications between the processor(s) 14 and the various components of the imaging system 10, e.g. any of the components of FIG. 1. Further, the communications module 26 may include a sensor interface 28 (e.g., one or more analog-to-digital converters) to permit signals transmitted from one or more probes (e.g. the ultrasound transducer 30) to be converted into signals that can be understood and processed by the processor(s) 14. It should be appreciated that the ultrasound transducer 30 may be communicatively coupled to the communications module 26 using any suitable means. For example, as shown in FIG. 2, the ultrasound transducer 30 may be coupled to the sensor interface 28 via a wired connection. However, in other embodiments, the ultrasound transducer 30 may be coupled to the sensor interface 28 via a wireless connection, such as by using any suitable wireless communications protocol known in the art. As such, the processor(s) 14 may be configured to receive one or more signals from the ultrasound transducer 30.

As used herein, the term "processor" refers not only to integrated circuits referred to in the art as being included in a computer, but also refers to a controller, a microcontroller, a microcomputer, a programmable logic controller (PLC), an application specific integrated circuit, a field-programmable gate array (FPGA), and other programmable circuits. The processor(s) 14 is also configured to compute advanced control algorithms and communicate to a variety of Ethernet or serial-based protocols (Modbus, OPC, CAN, etc.). Furthermore, in certain embodiments, the processor(s) 14 may communicate with a server through the Internet for cloud computing in order to reduce the computation time and burden on the local device. Additionally, the memory device(s) 16 may generally comprise memory element(s) including, but not limited to, computer readable medium (e.g., random access memory (RAM)), computer readable non-volatile medium (e.g., a flash memory), a floppy disk, a compact disc-read only memory (CD-ROM), a magneto-optical disk (MOD), a digital versatile disc (DVD) and/or other suitable memory elements. Such memory device(s) 16 may generally be configured to store suitable computer-readable instructions that, when implemented by the processor(s) 14, configure the processor(s) 14 to perform the various functions as described herein.

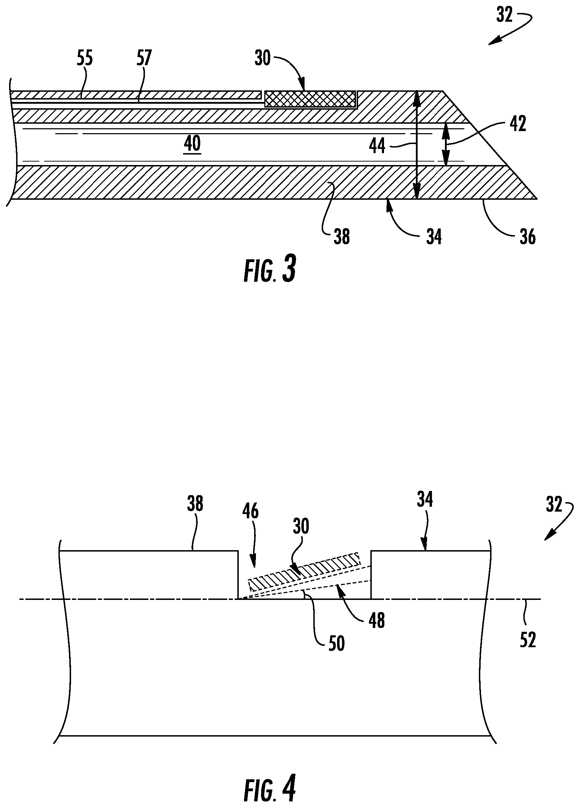

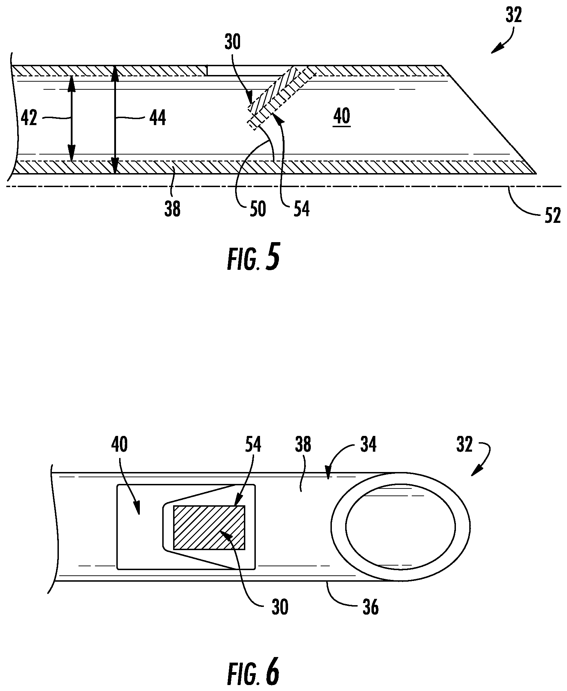

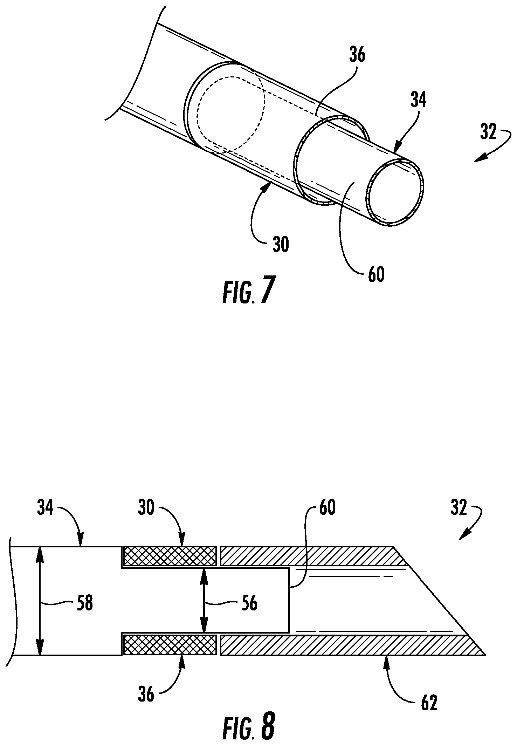

Referring now to FIGS. 3-8, various embodiments of a needle assembly 32 for the ultrasound imaging system 10 are illustrated. The embodiments shown in FIGS. 3 and 5-8 do not fall within the wording of the claims but help to illustrate features of the invention in accordance with the embodiment shown in FIG. 4. More specifically, as shown, the needle assembly 32 includes a needle 34 and the ultrasound transducer 30 mounted thereto. The ultrasound transducer 30 described herein may have any suitable configuration. For example, as shown in FIGS. 3-6, the ultrasound transducer 30 corresponds to a flat unidirectional transducer. Alternatively, as shown in FIGS. 7 and 8, the ultrasound transducer 30 may correspond to a cylindrical transducer. Further, as shown, the needle 34 defines a lumen 40 from a proximal end (not shown) to a distal end 36 thereof. In addition, as shown particularly in FIGS. 3 and 5, the needle 34 includes an outer wall 38 defined by an inner diameter 42 and an outer diameter 44. Moreover, as shown, the ultrasound transducer 30 is mounted to the outer wall 38 of the needle 34 at the distal end 36.

More specifically, as shown in the embodiment of FIGS. 3 and 4, the ultrasound transducer 30 is mounted within a cavity 46 defined within the outer wall 38 of the needle 34. The cavity 46 includes a bottom surface 48 defining a predetermined angle 50 with respect to a longitudinal axis 52 of the needle 34. As such, the ultrasound transducer 30 is configured to sit atop the bottom surface 48 of the cavity 46 at the predetermined angle 50. In addition, in certain embodiments, the predetermined angle 50 may be adjustable. For example, as shown, the predetermined angle 50 may be adjusted as shown by the dotted lines. Thus, the predetermined angle 50 described herein can be chosen to change and/or maximize the signal return and receive from the ultrasound transducer 30.

In additional embodiments, where the transducer 30 includes one or more wires 57 connected to the sensor interface 28, the outer wall 38 may also include one or more grooves 55 for receiving the wire(s) 57. In such embodiments, as shown, the groove(s) 55 may be embedded within the outer wall 38 of the needle 34.

Referring particularly to FIGS. 5 and 6, in an example outside the wording of the claims, the ultrasound transducer 30 may be mounted to a flap 54 formed with the outer wall 38 of the needle 34. **In** such embodiments, as shown, the flap 54 extends within the lumen 40 of the needle 34. Further and similar to the embodiment of FIGS. 3 and 4, the flap 54 may be positioned at the predetermined angle 50 with respect to the longitudinal axis 52 of the needle 34. As such, the ultrasound transducer 30 is configured to sit atop the flap 54 at the predetermined angle 50. **In** addition, in certain embodiments, the predetermined angle 50 may be adjustable. Further, as shown, the predetermined angle 50 may be limited by the outer wall 38 of the needle 34.

Referring now to FIGS. 7 and 8, which is a further example not falling within the scope of the invention, where in the ultrasound transducer 30 corresponds to a cylindrical transducer, the distal end 36 of the needle 34 may include a small outer diameter 56 than the diameter 58 at the proximal end. Thus, as shown particularly in FIG. 8, the cylindrical transducer 30 fits around the small outer diameter 56 of the distal end 36 of the needle 34. In further embodiments, a portion 60 of the distal end 36 of the needle 34 may extend beyond the cylindrical transducer 30. In such embodiments, as shown in FIG. 8, the needle assembly 32 may include a needle tip 62 secured to the portion 60 of the distal end 36 of the needle 34 that extends beyond the cylindrical transducer 30. For example, in one embodiment, the needle tip 62 may be secured to the distal portion 60 of the needle 34 via laser or spot welding. Such a configuration secures the transducer 30 to the needle 34.

In accordance with the invention, as generally shown in the figures, the ultrasound transducer 30 does not increase the outer diameter 44 of the needle 34. As such, the needle 34 and the ultrasound transducer 30 can be easily inserted into a patient.

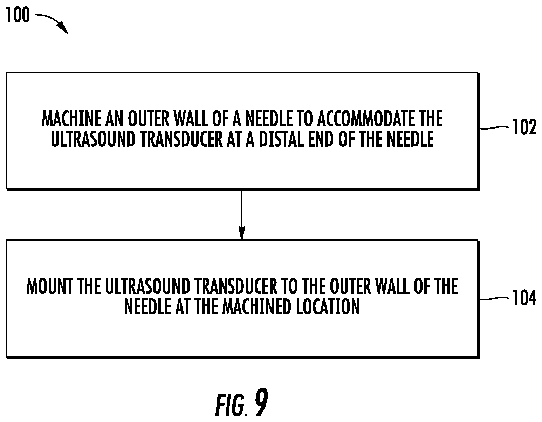

Referring now to FIG. 9, a flow diagram of one embodiment of a method 100 for mounting the ultrasound transducer 30 of the ultrasound imaging system 10 on the needle 34 to be inserted into a patient is illustrated. As shown at 102, the method 100 includes machining the outer wall 38 of the needle 34 to accommodate the ultrasound transducer 30 at the distal end 36 of the needle 34. As shown at 104, the method 100 includes mounting the ultrasound transducer 30 to the outer wall 36 of the needle 34 at the machined location.

The step of machining the outer wall 38 of the needle 34 to accommodate the ultrasound transducer 30 includes forming the cavity 46 (FIGS. 3 and 4) within the outer wall 38 of the needle and placing the ultrasound transducer into the cavity 46. The method 100 includes forming an angled bottom surface 48 in the cavity 46 and placing the ultrasound transducer 30 atop the angled bottom surface 48. In such embodiments, the method 100 may also include adjusting the angle 50 of the angled bottom surface 48.

In an example outside the wording of the claims, the step of machining the outer wall 38 of the needle 34 to accommodate the ultrasound transducer 30 may include forming the flap 54 in the outer wall 38 of the needle 34 and placing the ultrasound transducer 30 atop the flap 54. In still another embodiment, not falling within the scope of the invention, the step of machining the outer wall 38 of the needle 34 to accommodate the ultrasound transducer 30 may include removing a cylindrical portion of the outer wall 38 of the needle 34 (FIGS. 7 and 8) to decrease the outer diameter of the needle 34 at the distal end 36 thereof and placing the ultrasound transducer 30 around the reduced outer diameter 56.

In additional embodiments, the method 100 may include forming one or more grooves 55 in the outer wall 38 of the needle 34 and routing one or more wires 57 associated with the ultrasound transducer 30 through the groove(s) 55.

This written description uses examples to disclose the invention, including the best mode, and also to enable any person skilled in the art to practice the invention, including making and using any devices or systems and performing any incorporated methods. The patentable scope of the invention is defined by the claims.

## Claims

1. A needle assembly (32) for an ultrasound imaging system (10), the needle assembly (32) comprising:
a needle (34) suitable to be inserted into a patient, the needle (34) defining a lumen (40) from a proximal end to a distal end (36) thereof, the needle (34) comprising an outer wall (38) defined by an inner diameter and an outer diameter; and,
an ultrasound transducer (30) mounted to the outer wall (38) of the needle (34) at the distal end, wherein the ultrasound transducer (30) does not increase the outer diameter of the needle (34),
wherein a cavity (46) is defined within the outer wall (38) of the needle (34), **characterized in that** the cavity (46) comprises an angled bottom surface (48) defining a predetermined angle (50) with respect to a longitudinal axis (54) of the needle (34), and the ultrasound transducer (30) is mounted within the cavity (46) to sit atop the angled bottom surface (48).

2. The needle assembly of claim 1, wherein the predetermined angle (50) is adjustable.

3. The needle assembly of claim 1 or 2, wherein the ultrasound transducer (30) comprises a flat unidirectional transducer.

4. The needle assembly of any of the preceding claims, wherein the outer wall (38) comprises one or more grooves (55) for receiving one or more wires (57) associated with the ultrasound transducer (30).

5. The needle assembly of claim 4, wherein the one or more grooves (55) are embedded within the outer wall (38) of the needle (34).

6. A method for mounting an ultrasound transducer (30) of an ultrasound imaging system (10) on a needle (34) to be inserted into a patient, the method comprising:
machining an outer wall (38) of a needle (34) suitable to be inserted into a patient to accommodate the ultrasound transducer (30) at a distal end (36) of the needle (34); and,
mounting the ultrasound transducer (30) to the outer wall of the needle at the machined location such that the ultrasound transducer does not increase an outer diameter of the needle,
forming a cavity (46) within the outer wall (38) of the needle (34) **characterized by** forming an angled bottom surface (48) in the cavity (46), and placing the ultrasound transducer (30) into the cavity (46) atop the angled bottom surface (48).

7. The method of claim 6, further comprising adjusting an angle (50) of the angled bottom surface (48).

8. The method of claims 6 or 7, further comprising forming one or more grooves (55) in the outer wall (38) of the needle (34) and routing one or more wires (57) associated with the ultrasound transducer (30) through the one or more grooves (55).

## Patentansprüche

1. Nadelanordnung (32) für ein Ultraschallbildgebungssystem (10), wobei die Nadelanordnung (32) umfasst:
eine Nadel (34), die geeignet ist, um in einen Patienten eingeführt zu werden, wobei die Nadel (34) ein Lumen (40) von einem proximalen Ende bis zu einem distalen Ende (36) davon definiert, wobei die Nadel (34) eine Außenwand (38) umfasst, die durch einen Innendurchmesser und einen Außendurchmesser definiert ist; und
einen Ultraschallwandler (30), der an der Außenwand (38) der Nadel (34) am distalen Ende montiert ist, wobei der Ultraschallwandler (30) den Außendurchmesser der Nadel (34) nicht vergrößert,
wobei
ein Hohlraum (46) innerhalb der Außenwand (38) der Nadel (34) definiert ist, **dadurch gekennzeichnet, dass** der Hohlraum (46) eine abgewinkelte Bodenoberfläche (48) umfasst, die einen vorbestimmten Winkel (50) in Bezug auf eine Längsachse (54) der Nadel (34) definiert, und der Ultraschallwandler (30) innerhalb des Hohlraums (46) montiert ist, um auf der abgewinkelten Bodenoberfläche (48) zu sitzen.

2. Nadelanordnung nach Anspruch 1, wobei der vorbestimmte Winkel (50) anpassbar ist.

3. Nadelanordnung nach Anspruch 1 oder 2, wobei der Ultraschallwandler (30) einen flachen unidirektionalen Wandler umfasst.

4. Nadelanordnung nach einem der vorstehenden Ansprüche, wobei die Außenwand (38) eine oder mehrere Nuten (55) umfasst, um einen oder mehrere Drähte (57) aufzunehmen, die dem Ultraschallwandler (30) zugeordnet sind.

5. Nadelanordnung nach Anspruch 4, wobei die eine oder mehrere Nuten (55) in die Außenwand (38) der Nadel (34) eingebettet sind.

6. Verfahren zum Montieren eines Ultraschallwandlers (30) eines Ultraschallbildgebungssystems (10) an einer in einen Patienten einzuführenden Nadel (34), wobei das Verfahren umfasst:
Bearbeiten einer Außenwand (38) einer Nadel (34), die geeignet ist, um in einen Patienten eingeführt zu werden, um den Ultraschallwandler (30) an einem distalen Ende (36) der Nadel (34) unterzubringen; und
Montieren des Ultraschallwandlers (30) an der Außenwand der Nadel an der bearbeiteten Stelle, sodass der Ultraschallwandler einen Außendurchmesser der Nadel nicht vergrößert,
Bilden eines Hohlraums (46) innerhalb der Außenwand (38) der Nadel (34), **gekennzeichnet durch** Bilden einer abgewinkelten Bodenoberfläche (48) in dem Hohlraum (46) und Platzieren des Ultraschallwandlers (30) in dem Hohlraum (46) auf der abgewinkelten Bodenoberfläche (48).

7. Verfahren nach Anspruch 6, das weiter Anpassen eines Winkels (50) der abgewinkelten Bodenoberfläche (48) umfasst.

8. Verfahren nach Anspruch 6 oder 7, das weiter Bilden einer oder mehrerer Nuten (55) in der Außenwand (38) der Nadel (34) und Führen eines oder mehrerer Drähte (57), die dem Ultraschallwandler (30) zugeordnet sind, durch die eine oder mehrere Nuten (55) umfasst.

## Revendications

1. Ensemble aiguille (32) pour un système d'imagerie ultrasonore (10), l'ensemble aiguille (32) comprenant :
une aiguille (34) adaptée à l'insertion dans un patient, l'aiguille (34) définissant une lumière (40) d'une extrémité proximale à une extrémité distale (36) de celle-ci, l'aiguille (34) comprenant une paroi externe (38) définie par un diamètre intérieur et un diamètre extérieur ; et,
un transducteur ultrasonore (30) monté sur la paroi externe (38) de l'aiguille (34) à l'extrémité distale, dans lequel le transducteur ultrasonore (30) n'augmente pas le diamètre extérieur de l'aiguille (34),
dans lequel
une cavité (46) est définie dans la paroi externe (38) de l'aiguille (34), **caractérisé en ce que** la cavité (46) comprend une surface inférieure inclinée (48) définissant un angle prédéterminé (50) par rapport à un axe longitudinal (54) de l'aiguille (34), et le transducteur ultrasonore (30) est monté dans la cavité (46) pour reposer sur la surface inférieure inclinée (48).

2. Ensemble aiguille selon la revendication 1, dans lequel l'angle prédéterminé (50) est réglable.

3. Ensemble aiguille selon la revendication 1 ou 2, dans lequel le transducteur ultrasonore (30) comprend un transducteur unidirectionnel plat.

4. Ensemble aiguille selon l'une quelconque des revendications précédentes, dans lequel la paroi externe (38) comprend une ou plusieurs rainures (55) pour recevoir un ou plusieurs fils (57) associés au transducteur ultrasonore (30).

5. Ensemble aiguille selon la revendication 4, dans lequel les une ou plusieurs rainures (55) sont intégrées dans la paroi externe (38) de l'aiguille (34).

6. Procédé de montage d'un transducteur ultrasonore (30) d'un système d'imagerie ultrasonore (10) sur une aiguille (34) destinée à être insérée dans un patient, le procédé comprenant :
l'usinage d'une paroi externe (38) d'une aiguille (34) adaptée à l'insertion dans un patient pour loger le transducteur ultrasonore (30) à une extrémité distale (36) de l'aiguille (34) ; et,
le montage du transducteur ultrasonore (30) sur la paroi externe de l'aiguille à l'emplacement usiné de telle sorte que le transducteur ultrasonore n'augmente pas le diamètre extérieur de l'aiguille,
la formation d'une cavité (46) dans la paroi externe (38) de l'aiguille (34), **caractérisé par** la formation d'une surface inférieure inclinée (48) dans la cavité (46), et le placement du transducteur ultrasonore (30) dans la cavité (46) sur la surface inférieure inclinée (48).

7. Procédé selon la revendication 6, comprenant en outre le réglage d'un angle (50) de la surface inférieure inclinée (48).

8. Procédé selon les revendications 6 ou 7, comprenant en outre la formation d'une ou plusieurs rainures (55) dans la paroi externe (38) de l'aiguille (34) et l'acheminement d'un ou plusieurs fils (57) associés au transducteur ultrasonore (30) à travers les une ou plusieurs rainures (55).
